# EUROPÄISCHE PATENTSCHRIFT

(11) **EP 0 484 790 B1**
(45) Veröffentlichungstag und Bekanntmachung des Hinweises auf die Patenterteilung: **14.09.1994**
(21) Anmeldenummer: 91118375.4
(22) Anmeldetag: 29.10.1991
(51) Int. Cl.: C07C 209/60

(54) **Verfahren zur Herstellung von N-Methyl-alkylaminen**
Process for the preparation of N-methyl-alkylamines
Procédé pour la préparation de N-methyl-alkylamines

(30) Priorität: 07.11.1990 DE 4035307
(43) Veröffentlichungstag der Anmeldung: 13.05.1992
(73) Patentinhaber: HOECHST AKTIENGESELLSCHAFT, 65926 Frankfurt am Main (DE)
(72) Erfinder: Deymann, Detlef, W-4300 Essen (DE); Kampmann, Detlef, Dr. Dipl.-Chem., W-4630 Bochum (DE); Kniep, Claus, W-4200 Oberhausen (DE); Weber, Jürgen, Dr. Dipl.-Chem., W-4200 Oberhausen (DE)

(56) Entgegenhaltungen:
- FR-A- 1 525 149

## Beschreibung

Die vorliegende Erfindung betrifft ein Verfahren zur Herstellung von N-Methyl-alkylaminen der allgemeinen Formel CH₃-NH-CH₂-R, worin R für einen aliphatischen Rest steht. Amine dieser Art besitzen aufgrund ihrer Eigenschaften für eine Reihe technischer Anwendungen Bedeutung. Sie dienen als wichtige Zwischenprodukte zur Herstellung von Pflanzenschutzmitteln, Pharmazeutika, Additiven, Antioxidantien, Korrosionsschutzmitteln und Katalysatoren zur Herstellung von Kunststoffen, beispielsweise Epoxidharzen und Polyurethanen.

Eine Herstellung der N-Methyl-alkylamine ausgehend von Methylamin und einem Aldehyd der Formel R-CHO oder von einem Amin der Formel R-CH₂-NH₂ und Formaldehyd und nachfolgende Hydrierung der hierbei entstandenen Reaktionsprodukte, führt nicht im gewünschten Maße zum Erfolg. In beiden Verfahrensvarianten bilden sich in der Stufe der Hydrierung infolge von Umalkylierungen zahlreiche Nebenprodukte.

So liefert das von Arthur C. Cope, Norman A. LeBel et al. in J. Am. Chem. Soc. 79, 4720-4729 (1957) beschriebene Herstellungsverfahren für N-Methyl-n-propylamin unter Verwendung eines Platinkatalysators bei der Hydrierung des aus Aldehyd und primärem Amin resultierenden Reaktionsproduktes lediglich eine Wertproduktausbeute von 25 % (vgl. Seite 4727, Table IV, 1. Zeile).

Die von Henry R. Henze und David D. Humphreys in J. Am. Chem. Soc. 64, 2878-2880 (1942) durch Umsetzung von Methylamin mit n-Butanal und einer nachfolgenden Hydrierung des daraus resultierenden Azomethins (Schiffsche Base) in Anwesenheit von Raney-Nickel als Katalysator durchgeführte Herstellung von N-Methyl-n-butylamin führt neben beträchtlichen Mengen N-Methyl-di-n-butylamin lediglich zu 26 % N-Methyl-n-butylamin (vgl. Table I auf Seite 2879, 1. Zeile).

Modifiziert man die Herstellung von N-Methyl-n-butylamin, indem man die Hydrierung des Reaktionsproduktes von Methylamin und n-Butanal, wie von Armiger H. Sommers and Sharon E. Aaland in J. Org. Chem. 21, 484-485 (1956) angegeben, mittels LiAlH₄ durchführt, so erhält man eine Ausbeute von 55 % (vgl. Seite 484, rechte Spalte, Table I, 1. Zeile).

Es besteht daher ein Bedarf nach einem Verfahren, das einerseits leicht zugängliche Ausgangsstoffe verwendet und sich andererseits im technischen Maßstab mit vertretbarem Aufwand durchführen läßt. Ferner soll das Verfahren zu einer Verminderung der unerwünschten Nebenprodukte und zugleich zu einer Erhöhung der Ausbeute an Wertprodukt führen.

Gelöst wird diese Aufgabe durch ein Verfahren zur Herstellung von Aminen der Formel CH₃-NH-CH₂-R, worin R für einen aliphatischen Rest mit 1 bis 3 Kohlenstoffatomen steht. Es ist dadurch gekennzeichnet, daß man einen Aldehyd der Formel R-CHO, worin R für einen aliphatischen Rest mit 1 bis 3 Kohlenstoffatomen steht, mit einem Amin der Formel R'-NH₂, worin R' für einen geradkettigen oder verzweigten aliphatischen Rest mit 6 bis 12 Kohlenstoffatomen steht, zu einer Schiffschen Base umsetzt, das Reaktionswasser abtrennt und die Schiffsche Base mit Methylamin und Wasserstoff in Gegenwart eines Hydrierkatalysators umsetzt.

Die Umsetzung des Aldehyds der Formel R-CHO mit dem Amin der Formel R'-NH₂ führt unter Bildung von Wasser zu der entsprechenden Schiffschen Base (Azomethin). Die Reaktion verläuft bereits bei vergleichsweise niedrigen Temperaturen, doch wendet man, um die Reaktionszeit zu begrenzen, üblicherweise Temperaturen von 20 bis 80, insbesondere 30 bis 70, bevorzugt 40 bis 60°C an. Auf die Anwendung eines zusätzlichen Lösungsmittels kann bei der Herstellung des Azomethins üblicherweise verzichtet werden. Das infolge der Reaktion zwischen dem Aldehyd und dem Amin gebildete Reaktionsgemisch fällt heterogen an. Die obere organische Phase enthält die Schiffsche Base, während sich in der unteren Phase das Reaktionswasser nahezu quantitativ abscheidet.

Der Zusatz einer begrenzten Menge eines Lösungsmittels empfiehlt sich nur dann, wenn die Abtrennung des Reaktionswassers nicht im gewünschten Umfange eintritt. Geeignete Lösungsmittel sind z.B. Toluol, Xylol und Cyclohexan.

Als Aldehyde finden Acetaldehyd, Propionaldehyd, Isobutyraldehyd und n-Butyraldehyd, insbesondere Acetaldehyd, Propionaldehyd und Butyraldehyd, bevorzugt Propionaldehyd Verwendung.

Als Amin der Formel R'-NH₂ eignen sich geradkettige oder verzweigte aliphatische Amine, worin R' für einen Rest mit 6 bis 12, insbesondere 7 bis 12, bevorzugt 8 bis 10 Kohlenstoffatomen steht. Geeignete Amine sind n-Hexyl-, n-Heptyl-, n-Octyl-, 2-Ethylhexyl-, n-Nonyl-, n-Decyl-, n-Undecyl- und n-Dodecylamin sowie i-Hexyl-, i-Heptyl-, i-Octyl-, i-Nonyl-, i-Decyl-, i-Undecyl- und i-Dodecylamin und Mischungen vorstehend genannter Amine. Die Amine lassen sich technisch durch Hydroformylierung eines entsprechenden Olefins und nachfolgender Umsetzung der gebildeten Aldehyde mit Ammoniak und Wasserstoff herstellen.

Die Bildung der Schiffschen Base erfordert gemäß der Stöchiometrie der Reaktion je Mol Aldehyd ein Mol Amin. Es ist jedoch vorteilhaft das Amin bezogen auf Aldehyd im Überschuß zu verwenden. Man setzt je Mol Aldehyd der Formel R-CHO 1,02 bis 2,0, insbesondere 1,05 bis 1,8, bevorzugt 1,1 bis 1,5 Mol Amin der Formel R'-NH₂ zu.

Die Umsetzung des Aldehyds mit dem Amin kann kontinuierlich oder diskontinuierlich durchgeführt werden. Die Abtrennung des Reaktionswassers kann entweder während der Reaktion oder nach Abschluß der Bildung des Azomethins bei Temperaturen von 10 bis 80, insbesondere 15 bis 50, bevorzugt 20 bis 35°C erfolgen.

Das aus dem Aldehyd der Formel R-CHO und dem Amin der Formel R'-NH₂ gebildete Azomethin wird anschließend mit Methylamin und Wasserstoff in Gegenwart eines Hydrierkatalysators umgesetzt. Die Reaktion verläuft besonders gut, wenn Methylamin in ausreichendem stöchiometrischem überschuß bezogen auf Schiffsche Base verwendet wird. Es empfiehlt sich je Mol Schiffsche Base 4 bis 40, insbesondere 5 bis 30, bevorzugt 10 bis 20 Mol Methylamin einzusetzen.

Um aus der Schiffschen Base das entsprechende N-Methylalkylamin herzustellen, benötigt man entsprechend der Stöchiometrie der Umsetzung je Mol Azomethin ein Mol Wasserstoff. Üblicherweise setzt man jedoch den Wasserstoff in einem ausreichenden Überschuß ein. Da der für die Umsetzung erforderliche erhöhte Druck durch die Zugabe von Wasserstoff eingestellt wird, gelangt auf diese Weise eine ausreichende Menge Wasserstoff in die Reaktion.

Man setzt die Schiffsche Base, Methylamin und Wasserstoff bei 80 bis 200, insbesondere 90 bis 180, bevorzugt 100 bis 160°C und 5 bis 25, insbesondere 8 bis 20, bevorzugt 10 bis 15 MPa um.

Ein weiterer Vorteil des erfindungsgemäßen Verfahrens besteht darin, daß es nicht auf den Gebrauch spezieller Katalysatoren beschränkt ist, sondern die Verwendung einer großen Anzahl üblicher Hydrierkatalysatoren ermöglicht. Der Katalysator kann in stückiger Form oder als Suspension zum Einsatz gelangen.

Die Katalysatoren können Trägerstoffe besitzen, aber auch frei von Trägern sein. Sie enthalten Ni, Co, Cu, Mn, Fe, Rh, Pt und/oder Pd, insbesondere Ni, Co, Cu und/oder Pd, bevorzugt Ni, Co und/oder Pd und daneben gegebenenfalls gebräuchliche Zusatzstoffe und Promotoren, beispielsweise Erdalkalioxide, SiO₂, Al₂O₃, MnO₂ und/oder Cr₂O₃.

Vorteilhaft ist der Gebrauch von Trägerkatalysatoren. Als Träger kommen Al₂O₃, SiO₂, Kieselgel, Kieselerde, Aktivkohle und/oder Bimsstein, insbesondere SiO₂, Kieselgel, Kieselerde und/oder Aktivkohle in Betracht.

Die Trägerkatalysatoren enthalten üblicherweise 10 bis 75, insbesondere 20 bis 70, bevorzugt 40 bis 65 Gew.-% Ni, Co, Cu, Mn und/oder Fe bezogen auf gesamte Katalysatormasse. Besonders bewährt haben sich Katalysatoren mit 20 bis 70, insbesondere 40 bis 65 Gew.-% Ni und/oder Co bezogen auf gesamte Katalysatormasse.

Die für die Umsetzung geeigneten Edelmetallkatalysatoren sind üblicherweise auf einem Träger untergebracht und weisen eine Metallgehalt von 0,1 bis 20, insbesondere 0,2 bis 15, bevorzugt 0,5 bis 10 Gew.-% bezogen auf gesamte Katalysatormasse auf. Geeignete Edelmetalle sind Rh, Pt und/oder Pd, insbesondere Pt und/oder Pd, bevorzugt Pd. Als Träger empfehlen sich geformte Materialien auf Basis von Al₂O₃, SiO₂, Aktivkohle, Kieselgel, Kieselgur und/oder Bimsstein, insbesondere Al₂O₃, SiO₂, Kieselgel, Kieselgur und/oder Aktivkohle, bevorzugt Kieselgel, Kieselgur und/oder Aktivkohle.

Schiffsche Base, Methylamin und Wasserstoff werden innig vermischt in Gegenwart des Hydrierkatalysators umgesetzt. Die Reaktion kann diskontinuierlich oder kontinuierlich durchgeführt werden.

Besonders einfach gestaltet sich eine diskontinuierliche Durchführung der Umsetzung unter Verwendung eines suspendierten Hydrierkatalysators. Man legt in einem druckfesten Behälter Schiffsche Base, Methylamin und Katalysator vor, preßt Wasserstoff auf und erwärmt unter Rühren. Sobald die Aufnahme von Wasserstoff abgeschlossen ist, ist die Reaktion beendet. Nachfolgend wird der Katalysator beispielsweise durch Filtration und/oder Zentrifugieren abgetrennt. Das verbleibende Reaktionsgemisch kann anschließend destillativ aufgearbeitet werden.

Besonders einfach gestaltet sich die kontinuierliche Umsetzung der Schiffschen Base mit Methylamin und Wasserstoff unter Verwendung druckfester Rohrreaktoren, die den Hydrierkatalysator in stückiger Form angeordnet als Festbett enthalten. Die Einsatzstoffe werden dem Reaktor entweder am Kopf oder am Boden zugeführt. Je nach Zugabeart spricht man von Riesel- oder Sumpffahrweise. Bei Anwendung der Rieselfahrweise verläßt das Reaktionsprodukt den Reaktor am Boden und bei Ausübung der Sumpffahrweise tritt das Reaktionsgemisch am Kopf des Reaktors aus. Falls gewünscht kann auch ein Teil des Reaktionsgemisches wieder in den Reaktor zurückgeführt werden.

Das Verfahren ist besonders geeignet zur Herstellung von Methyl-n-propylamin. Ein besonders geeignetes Amin ist 2-Ethylhexylamin.

Die nachfolgenden Beispiele belegen die Erfindung, ohne sie zu begrenzen.

### Experimenteller Teil

### Beispiel 1: Herstellung von N-Methyl-n-propylamin

In einem mit Rührer, Thermometer, Tropftrichter und Rückflußkühler bestückten Dreihalskolben (Volumen 4 l) werden 1 350 g (10,5 Mol) 2-Ethylhexylamin vorgelegt. Unter Rühren tropft man innerhalb von 2 Stunden 551 g (9,5 Mol) Propanal in einer Geschwindigkeit zu, daß eine Temperatur von 45 bis 50°C eingehalten wird. Nach Ende der Propanalzugabe wird noch 2 Stunden gerührt, wobei die Temperatur allmählich wieder auf Raumtemperatur absinkt. Man beendet das Rühren. Es bilden sich zwei Phasen, deren Trennung sich problemlos durchführen läßt.

Die untere wasserhaltige Phase umfaßt 157 g (entsprechend 92 % der theoretischen Menge) Reaktionswasser. Die obere organische Phase, die das aus Propanal und 2-Ethylhexylamin gebildete Azomethin (Schiffsche Base) enthält, wird in einen Autoklav überführt und in Gegenwart von 5 Gew.-% eines Nickelkatalysators, der 50 bis 53 Gew.-% Ni und etwa 25 bis 30 Gew.-% Kieselgur als Träger enthält, mit Methylamin umgesetzt. Je Mol Azomethin werden 10 Mol Methylamin eingesetzt.

Die Umsetzung erfolgt bei 140°C und 10 MPa über einen Zeitraum von 3 Stunden. Die gaschromatografische Analyse des anfallenden Reaktionsgemisches weist, ohne überschüssig eingesetztes Methylamin und freigesetztes 2-Ethylhexylamin zu berücksichtigen, folgende Zusammensetzung aus:

**Tabelle 1**

| Zusammensetzung des Reaktionsgemisches (Gaschromatografische Analyse ohne CH₃NH₂ und 2-Ethylhexylamin) | |
|---|---|
| Vorlauf | 5,4 Gew.-% |
| N-Methyl-n-propylamin | 73,6 Gew.-% |
| Zwischenlauf | 8,7 Gew.-% |
| N-Propyl-2-ethylhexylamin | 12,3 Gew.-% |

Durch Destillation (Normaldruck, Kolonne mit 24 theor. Böden) läßt sich N-Methyl-n-propylamin in einer Reinheit > 99 % gewinnen. Das zurückgewonnene 2-Ethylhexylamin kann in die Synthese (Azomethin-Bildung) zurückgeführt werden.

### Vergleichsversuch 1: Herstellung von N-Methyl-n-propylamin

In einem mit Rührer, Thermometer, Tropftrichter und Rückflußkühler bestückten Dreihalskolben (Volumen 4 l) werden 815 g einer 40-%igen wäßrigen Methylaminlösung (entsprechend 10,5 Mol Methylamin) vorgelegt. Wie in Beispiel 1 angegeben tropft man innerhalb von 2 Stunden 551 g (9,5 Mol) Propanal zu, rührt nach Beendigung der Propanalzugabe noch 2 Stunden, trennt die wäßrige von der organischen Phase, überführt die obere, das aus Propanal und Methylamin gebildete Azomethin (Schiffsche Base) enthaltende Phase in einen Autoklav. Die Umsetzung erfolgt in Gegenwart von 5 Gew.-% des in Beispiel 1 verwendeten Katalysators bei 140°C und 10 MPa über einen Zeitraum von 3 Stunden. Die gaschromatografische Analyse des anfallenden Reaktionsgemisches weist, ohne Methylamin zu berücksichtigen, folgende Zusammensetzung aus:

**Tabelle 2**

| Zusammensetzung des Reaktionsgemisches (Gaschromatische Analyse ohne CH₃NH₂) | |
|---|---|
| Vorlauf | 0,2 Gew.-% |
| n-Propylamin | 1,1 Gew.-% |
| N-Methyl-n-propylamin | 11,2 Gew.-% |
| N,N-Dimethyl-n-propylamin | 1,8 Gew.-% |
| Di-n-propylamin | 0,6 Gew.-% |
| N-Methyl-di-n-propylamin | 5,1 Gew.-% |
| Höhersieder | 80,0 Gew.-% |

Durch Destillation (Normaldruck, Kolonne mit 24 theor. Böden) läßt sich N-Methyl-n-propylamin in einer Reinheit von lediglich 84 % gewinnen, da eine Abtrennung des durch Umalkylierung erhaltenen N,N-Dimethyl-n-propylamins infolge seines nahezu gleichen Siedepunktes nicht möglich ist.

### Beispiel 2: Herstellung von N-Methyl-ethylamin

Man arbeitet - ausgehend von 1 356 g (10,5 Mol) 2-Ethylhexylamin und 418 g (9,5 Mol) Acetaldehyd - genau wie in Beispiel 1 angegeben.

Die gaschromatografische Analyse des anfallenden Reaktionsgemisches weist, ohne überschüssig eingesetztes Methylamin und freigesetztes 2-Ethylhexylamin zu berücksichtigen, folgende Zusammensetzung aus:

**Tabelle 3**

| Zusammensetzung des Reaktionsgemisches (Gaschromatografische Analyse ohne CH₃NH₂ und 2-Ethylhexylamin) | |
|---|---|
| Vorlauf | 1,5 Gew.-% |
| N-Methyl-ethylamin | 80,8 Gew.-% |
| N-Methyl-n-butylamin | 0,8 Gew.-% |
| 2,4,6-Trimethyl-1,3,5-trioxan | 4,6 Gew.-% |
| N-2-Ethylhexyl-ethylamin | 12,3 Gew.-% |

### Vergleichsversuch 2: Herstellung von N-Methyl-ethylamin

Man arbeitet wie in Vergleichsversuch 1 angegeben, setzt jedoch statt Propanal 418 g (9,5 Mol) Acetaldehyd ein.

Die gaschromatografische Analyse des anfallenden Reaktionsgemisches weist, ohne Methylamin zu berücksichtigen, folgende Zusammensetzung aus:

**Tabelle 4**

| Zusammensetzung des Reaktionsgemisches (Gaschromatografische Analyse ohne CH₃NH₂) | |
|---|---|
| Vorlauf | 1,5 Gew.-% |
| N-Methyl-ethylamin | 34,5 Gew.-% |
| N,N-Diethyl-methylamin | 0,9 Gew.-% |
| N-Methyl-n-butylamin | 13,8 Gew.-% |
| 2,4,6-Trimethyl-1,3,5-trioxan | 4,3 Gew.-% |
| N-Ethyl-N-methyl-n-butylamin | 7,2 Gew.-% |
| Höhersieder | 37,8 Gew.-% |

### Beispiel 3: Herstellung von N-Methyl-n-butylamin

Man arbeitet - ausgehend von 1 356 g (10,5 Mol) 2-Ethylhexylamin und 685 g (9,5 Mol) n-Butanal - genau wie in Beispiel 1 angegeben.

Die gaschromatografische Analyse des anfallenden Reaktionsgemisches weist, ohne überschüssig eingesetztes Methylamin und freigesetztes 2-Ethylhexylamin zu berücksichtigen, folgende Zusammensetzung aus:

**Tabelle 5**

| Zusammensetzung des Reaktionsgemisches (Gaschromatografische Analyse ohne CH₃NH₂ und 2-Ethylhexylamin) | |
|---|---|
| Vorlauf | 0,6 Gew.-% |
| Komponente | 7,3 Gew.-% |
| Zwischenlauf | 0,3 Gew.-% |
| N-Methyl-n-butylamin | 82,3 Gew.-% |
| Höhersieder | 9,5 Gew.-% |

### Vergleichsversuch 3: Herstellung von N-Methyl-n-butylamin

Man arbeitet wie in Vergleichsversuch 1 angegeben, setzt jedoch statt Propanal 685 g (9,5 Mol) n-Butanal ein.

Die gaschromatografische Analyse des anfallenden Reaktionsgemisches weist, ohne Methylamin zu berücksichtigen, folgende Zusammensetzung aus:

**Tabelle 6**

| Zusammensetzung des Reaktionsgemisches (Gaschromatografische Analyse ohne CH₃NH₂) | |
|---|---|
| Vorlauf | 2,0 Gew.-% |
| N-Methyl-n-butylamin | 71,5 Gew.-% |
| N,N-Dimethyl-n-butylamin | 1,0 Gew.-% |
| N,N-Di-n-butyl-methylamin | 9,3 Gew.-% |
| N-Methyl-2-ethylhexylamin | 6,0 Gew.-% |
| N,N-Di-n-butyl-2-ethylhexylamin | 6,2 Gew.-% |
| Höhersieder | 4,0 Gew.-% |

## Patentansprüche

1. Verfahren zur Herstellung von Aminen der Formel CH₃-NH-CH₂-R, worin R für einen aliphatischen Rest mit 1 bis 3 Kohlenstoffatomen steht, dadurch gekennzeichnet, daß man einen Aldehyd der Formel R-CHO, worin R für einen aliphatischen Rest mit 1 bis 3 Kohlenstoffatomen steht, mit einem Amin der Formel R'-NH₂, worin R' für einen geradkettigen oder verzweigten aliphatischen Rest mit 6 bis 12 Kohlenstoffatomen steht, zu einer Schiffschen Base umsetzt, das Reaktionswasser abtrennt und die Schiffsche Base mit Methylamin und Wasserstoff in Gegenwart eines Hydrierkatalysators umsetzt.

2. Verfahren nach Anspruch 1, dadurch gekennzeichnet, daß man den Aldehyd mit dem Amin der Formel R'-NH₂ bei 20 bis 80, insbesondere 30 bis 70, bevorzugt 40 bis 60°C umsetzt.

3. Verfahren nach einem oder mehreren der Ansprüche 1 bis 2, dadurch gekennzeichnet, daß man je Mol Aldehyd 1,02 bis 2,0, insbesondere 1,05 bis 1,8, bevorzugt 1,1 bis 1,5 Mol Amin einsetzt.

4. Verfahren nach Anspruch 1 bis 3, dadurch gekennzeichnet, daß je Mol Schiffsche Base 4 bis 40, insbesondere 5 bis 30, bevorzugt 10 bis 20 Mol Methylamin eingesetzt werden.

5. Verfahren nach einem oder mehreren der Ansprüche 1 bis 4, dadurch gekennzeichnet, daß man die Schiffsche Base mit Methylamin und Wasserstoff bei 80 bis 200, insbesondere 90 bis 180, bevorzugt 100 bis 160°C und 5 bis 25, insbesondere 8 bis 20, bevorzugt 10 bis 15 MPa umsetzt.

6. Verfahren nach einem oder mehreren der Ansprüche 1 bis 5, dadurch gekennzeichnet, daß man die Schiffsche Base in Gegenwart eines Ni, Co, Cu, Mn, Fe, Rh, Pt, und/oder Pd, insbesondere Ni, Co, Cu und/oder Pd, bevorzugt Ni, Co und/oder Pd enthaltenden Hydrierkatalysators mit Methylamin und Wasserstoff umsetzt.

7. Verfahren nach einem oder mehreren der Ansprüche 1 bis 6, dadurch gekennzeichnet, daß der Hydrierkatalysator 20 bis 70, insbesondere 40 bis 65 Gew.-% Nickel, bezogen auf Hydrierkatalysator, enthält.

8. Verfahren nach einem oder mehreren der Ansprüche 1 bis 7, dadurch gekennzeichnet, daß man als Aldehyd Propionaldehyd einsetzt.

9. Verfahren nach einem oder mehreren der Ansprüche 1 bis 8, dadurch gekennzeichnet, daß man als Amin n-Hexyl-, n-Heptyl-, n-Octyl-, 2-Ethylhexyl-, n-Nonyl-, n-Decyl-, n-Undecyl- und n-Dodecylamin sowie i-Hexyl-, i-Heptyl-, i-Octyl-, i-Nonyl-, i-Decyl-, i-Undecyl- und i-Dodecylamin und Mischungen derselben, insbesondere 2-Ethylhexylamin einsetzt.

## Claims

1. A process for the preparation of an amine of the formula CH₃-NH-CH₂-R in which R is an aliphatic radical having 1 to 3 carbon atoms, which comprises reacting an aldehyde of the formula R-CHO in which R is an aliphatic radical having 1 to 3 carbon atoms, with an amine of the formula R'-NH₂ in which R' is a straight-chain or branched aliphatic radical having 6 to 12 carbon atoms, to give a Schiff base, removing the water of reaction, and reacting the Schiff base with methylamine and hydrogen in the presence of a hydrogenation catalyst.

2. The process as claimed in claim 1, wherein the aldehyde is reacted with the amine of the formula R'-NH₂ at from 20 to 80°C, in particular from 30 to 70°C, preferably from 40 to 60°C.

3. The process as claimed in one or more of claims 1 and 2, wherein from 1.02 to 2.0 mol, in particular from 1.05 to 1.8 mol, preferably from 1.1 to 1.5 mol, of amine are employed per mole of aldehyde.

4. The process as claimed in any of claims 1 to 3, wherein from 4 to 40 mol, in particular from 5 to 30 mol, preferably from 10 to 20 mol, of methylamine are employed per mole of Schiff base.

5. The process as claimed in one or more of claims 1 to 4, wherein the Schiff base is reacted with methylamine and hydrogen at from 80 to 200°C, in particular from 90 to 180°C, preferably from 100 to 160°C, and at from 5 to 25 MPa, in particular from 8 to 20 MPa, preferably from 10 to 15 MPa.

6. The process as claimed in one or more of claims 1 to 5, wherein the Schiff base is reacted with methylamine and hydrogen in the presence of a hydrogenation catalyst containing Ni, Co, Cu, Mn, Fe, Rh, Pt and/or Pd, in particular Ni, Co, Cu and/or Pd, preferably Ni, Co and/or Pd.

7. The process as claimed in one or more of claims 1 to 6, wherein the hydrogenation catalyst contains from 20 to 70 % by weight, in particular from 40 to 65 % by weight, of nickel, based on the hydrogenation catalyst.

8. The process as claimed in one or more of claims 1 to 7, wherein the aldehyde employed is propionaldehyde.

9. The process as claimed in one or more of claims 1 to 8, wherein the amine employed is n-hexylamine, n-heptylamine, n-octylamine, 2-ethylhexylamine, n-nonylamine, n-decylamine, n-undecylamine, n-dodecylamine, i-hexylamine, i-heptylamine, i-octylamine, i-nonylamine, i-decylamine, i-undecylamine or i-dodecylamine, or a mixture thereof, in particular 2-ethylhexylamine.

## Revendications

1. Procédé de préparation d'amines de formule CH₃-NH-CH₂-R dans laquelle R représente un radical aliphatique en C₁-C₃, caractérisé en ce que l'on fait réagir un aldéhyde de formule R-CHO dans laquelle R représente un radical aliphatique en C₁-C₃ avec une amine de formule R'-NH₂ dans laquelle R' représente un radical aliphatique à chaîne droite ou ramifiée en C₆-C₁₂, la réaction donnant une base de Schiff d'où l'on sépare l'eau formée dans la réaction et qu'on fait réagir avec la méthylamine et l'hydrogène en présence d'un catalyseur d'hydrogénation.

2. Procédé selon la revendication 1, caractérisé en ce que l'on fait réagir l'aldéhyde avec l'amine de formule R'-NH₂ à des températures de 20 à 80, plus spécialement de 30 à 70 et de préférence de 40 à 60°C.

3. Procédé selon une ou plusieurs des revendications 1 et 2, caractérisé en ce que l'on utilise de 1,02 à 2,0, plus spécialement de 1,05 à 1,8 et de préférence de 1,1 à 1,5 mole de l'amine par mole de l'aldéhyde.

4. Procédé selon les revendications 1 à 3, caractérisé en ce que l'on utilise de 4 à 10 moles, plus spécialement de 5 à 30 moles et de préférence de 10 à 20 moles de méthylamine par mole de la base de Schiff.

5. Procédé selon une ou plusieurs des revendications 1 à 4, caractérisé en ce que l'on fait réagir la base de Schiff avec la méthylamine et l'hydrogène à des températures de 80 à 200, plus spécialement de 90 à 180, de préférence de 100 à 160°C sous des pressions de 5 à 25, plus spécialement de 8 à 20 et de préférence de 10 à 15 MPa.

6. Procédé selon un ou plusieurs des revendications 1 à 5, caractérisé en ce que l'on fait réagir la base de Schiff avec la méthylamine et l'hydrogène en présence d'un catalyseur d'hydrogénation contenant Ni, Co, Cu, Mn, Fe, Rh, Pt et/ou Pd, plus spécialement Ni, Co, Cu et/ou Pd, de préférence Ni, Co et/ou Pd.

7. Procédé selon une ou plusieurs des revendications 1 à 6, caractérisé en ce que le catalyseur d'hydrogénation contient de 20 à 70, plus spécialement de 40 à 65 % en poids de nickel sur son poids total.

8. Procédé selon une ou plusieurs des revendications 1 à 7, caractérisé en ce que l'aldéhyde utilisé est le propionaldéhyde.

9. Procédé selon une ou plusieurs des revendications 1 à 8, caractérisé en ce que l'amine utilisée est la n-hexyl-, la n-heptyl-, la n-octyl-, la 2-éthylhexyl-, la n-nonyl-, la n-décyl-, la n-undécyl- ou la n-dodécyl-amine ou encore l'iso-hexyl-, l'iso-heptyl-, l'iso-octyl-, l'iso-nonyl-, l'iso-décyl-, l'iso-undécyl- ou l'iso-dodécylamine ou un mélange de ces amines, et en particulier la 2-éthylhexylamine.
